(19) Europäisches Patentamt
European Patent Office
Office européen des brevets

(11) **EP 0 790 055 B1**

(12) **FASCICULE DE BREVET EUROPEEN**

(45) Date de publication et mention
de la délivrance du brevet:
**03.05.2000 Bulletin 2000/18**

(51) Int Cl.⁷: **A61K 7/48**, A61K 7/02

(21) Numéro de dépôt: **97400219.8**

(22) Date de dépôt: **30.01.1997**

(54) **Utilisation d'un organopolysiloxane solide élastomère associé à une phase grasse pour la préparation d'une composition ou dans une composition de soin ou de maquillage pour matifier la peau**

Verwendung von einem festen elastomerischen Organopolysiloxan in einer Fettphase zur Herstellung einer Zusammensetzung oder in einem Make-up oder Hautpflegemittel mit mattem Aussehen

Use of a solid elastomeric organopolysiloxane in a fatty phase for the preparation of a composition or in a make-up or skin care composition for giving a matt appearance to the skin

(84) Etats contractants désignés:
**AT BE CH DE ES FR GB IT LI NL SE**

(30) Priorité: **19.02.1996 FR 9602021**

(43) Date de publication de la demande:
**20.08.1997 Bulletin 1997/34**

(73) Titulaire: **L'OREAL**
**75008 Paris (FR)**

(72) Inventeur: **Bara, Isabelle**
**75013 Paris (FR)**

(74) Mandataire: **Lhoste, Catherine**
**L'OREAL,**
**D.P.I.,**
**6, rue Bertrand Sincholle**
**92585 Clichy Cédex (FR)**

(56) Documents cités:
**EP-A- 0 293 795**          **EP-A- 0 381 166**
**WO-A-96/18374**

- **DATABASE WPI Week 8641 Derwent Publications Ltd., London, GB; AN 86-267848 XP002019603 "Make-up cosmetic composition contains cured organopolysiloxane powder" & JP 61 194 009 A (TORAY SILICONE KK) , 28 Août 1986**
- **PATENT ABSTRACTS OF JAPAN vol. 95, no. 010 & JP 07 258028 A (SHISEIDO CO LTD), 9 Octobre 1995,**
- **PATENT ABSTRACTS OF JAPAN vol. 95, no. 010 & JP 07 258027 A (SHISEIDO CO LTD), 9 Octobre 1995,**

**Description**

La présente demande concerne l'utilisation d'un organopolysiloxane solide élastomérique partiellement réticulé associé à une phase grasse pour la préparation d'une composition ou dans une composition de soin de la peau ou de maquillage pour matifier la peau ainsi que ses diverses applications.

Les compositions de soin de la peau ou de maquillage ayant des propriétés matifiantes sont généralement utilisées pour résoudre les problèmes de brillance occasionnés par un excès de sébum et pour améliorer la tenue du maquillage à long terme qui a tendance à se dégrader visuellement au cours de la journée. Elles donnent un aspect mat à la peau résultant d'un pouvoir diffusant de la lumière à la surface de la peau. Elles peuvent aussi être utilisées pour estomper les défauts de la peau tels que les microreliefs, les rides, les ridules, les pores ou les variations de couleur.

Les compositions classiques dites matifiantes contiennent généralement très peu de corps gras ou sont dépourvues de corps gras. Elles sont généralement constituées de poudres adsorbantes du sébum et d'huile excédentaire de la composition non-adsorbée par la peau. Parmi les poudres matifiantes d'origine naturelle ou synthétique, on peut citer notamment les charges telles que le talc, l'amidon, le mica , la silice, les poudres de Nylon, les poudres de polyéthylène, , la poly-béta-alanine, les poly(méthacrylate de méthyle). Ce type de charges présentent l'inconvénient de ne pas apporter à la peau un aspect naturel en donnant un aspect poudreux voire plâtreux et d'accentuer les défauts de la peau. De plus, les compositions les contenant sont généralement desséchantes à long terme et s'étalent difficilement. Leur effet matifiant est peu durable dans le temps.

On connaît également dans la demande EP-A-052 769 des compositions matifiantes apportant une couche translucide et un aspect naturel à la peau maquillée. Il s'agit de dispersions de particules sphériques dans un liant gras dans un rapport en poids charges/liant très spécifique. Ces compositions peuvent être desséchantes ; elles ont tendance à pelucher lors de l'étalement et à donner un effet blanchissant à la peau en raison d'une forte concentration en poudre.

On a proposé également des sérums réalisés à partir d'une phase plus ou moins gélifiante hydrosoluble dépourvue de phase grasse ou contenant une faible teneur en corps gras. Ces formulations ont tendance à être inconfortables pour la peau en raison de leur faible teneur en corps gras et de la présence de gélifiants conduisant à un effet collant et à un effet tenseur sur la peau.

La Demanderesse a découvert de manière surprenante que l'association d'un organopolysiloxane solide élastomérique partiellement réticulé avec une phase grasse pouvait constituer un agent matifiant remarquable.

La Demanderesse a découvert, en effet, qu'une telle association permettait d'obtenir des compositions pour le soin ou le maquillage ayant un bon pouvoir diffusant de la lumière en surface, stable dans le temps, conférant à la peau un aspect mat de façon prolongée dans le temps.

La Demanderesse à découvert également que cette association permettait également d'obtenir des produits de soin ou de maquillage permettant d'estomper les imperfections du relief de la peau tout en lui apportant un aspect naturel.

Les organopolysiloxanes élastomérique partiellement réticulés de l'invention associés à une phase grasse présentent un remarquable pouvoir gélifiant d'huile, ne sont pas desséchants pour la peau et apportent de bonnes propriétés cosmétiques. Ces nouveaux agents matifiants permettent de formuler des gels ou des crèmes confortables à l'application, doux, s'étalant facilement, non-collants au toucher et qui ne dessèchent pas à long terme.

Par « élastomérique », on entend un matériau souple, déformable ayant des propriétés viscoélastiques et présentant notamment la consistance d'une éponge ou d'une sphère souple.

Les organopolysiloxanes élastomérique partiellement réticulés conformes à l'invention sont en général partiellement ou totalement réticulés et de structure tridimensionnelle. Inclus dans une phase grasse, ils se transforment, selon le taux de phase grasse utilisé, d'un produit d'aspect spongieux lorsqu'ils sont utilisés en présence de faibles teneurs en phase grasse en un gel homogène, en présence de quantités de phase grasse plus élevées. La gélification de la phase grasse par ces élastomères peut être totale ou partielle.

Les agents matifiants de l'invention se présentent généralement sous forme de gel constitué d'un organopolysiloxane élastomérique partiellement réticulé de structure tridimensionnelle, inclus dans au moins une huile hydrocarbonée et/ou une huile siliconée.

Les organopolysiloxanes élastomérique partiellement réticulés selon l'invention constituant l'agent matifiant de l'invention peuvent être choisis parmi les polymères réticulés décrits dans la demande EP-A-0295886.

Selon cette demande, ils sont obtenus par réaction d'addition et de réticulation, en présence d'un catalyseur du type platine, d'au moins :

(a) un organopolysiloxane ayant au moins deux groupes alcényle inférieurs par molécule ; et
(b) un organopolysiloxane ayant au moins deux atomes d'hydrogène liés à un atome de silicium par molécule.

Les organopolysiloxanes élastomérique partiellement réticulés selon l'invention constituant l'agent matifiant peuvent être choisis parmi ceux décrits dans le brevet US 5.266.321.

Selon ce brevet, ils sont choisis notamment parmi :

i) les organopolysiloxanes comprenant des motifs $R_2SiO$ et $RSiO_{1,5}$ et éventuellement des motifs $R_3SiO_{0,5}$ et/ou $SiO_2$ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs $R_2SiO$ sur les motifs $RSiO_{1,5}$ varie de 1/1 à 30/1 ;

ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol quand l'organopolysiloxane est non-cyclique et entre 1 et 50% mol lorsque l'organopolysiloxane est cyclique.

Les organopolysiloxanes objet de l'invention sont par exemple ceux commercialisés sous les noms KSG6 de Shin-Etsu, Trefil E-505C ou Trefil E-506C de Dow-Corning, Gransil de Grant Industries (SR-CYC, SR DMF10, SR-DC556), ou ceux commercialisés sous forme de gels déjà constitués (KSG15, KSG17, KSG16, KSG18 de Shin-Etsu, Gransil SR 5CYC gel, Gransil SR DMF 10 gel, Gransil SR DC 556 gel, SF 1204 et JK 113 de General Electric. On peut aussi utiliser un mélange de ces produits commerciaux.

La phase grasse associée à l'organopolysiloxane solide élastomérique partiellement réticulé pour former un agent matifiant est constituée d'au moins une huile hydrocarbonée et/ou d'au moins une huile siliconée.

Les huiles hydrocarbonées utilisées selon l'invention en association avec l'organopolysiloxane élastomérique partiellement réticulé sont choisies parmi :

- les huiles d'origine animale telles que le perhydrosqualène ;
- les huiles végétales telles que les triglycérides liquides, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société STEARINERIES DUBOIS ou ceux vendus sous les dénominations MIGLYOL 810, 812 et 818 par la société DYNAMIT NOBEL ;
- les huiles de formule $R_9COOR_{10}$ dans laquelle $R_9$ représente le reste d'un acide gras supérieur comportant de 7 à 19 atomes de carbone et $R_{10}$ représente une chaîne hydrocarbonée ramifiée contenant de 3 à 20 atomes de carbone, par exemple, l'huile de Purcellin ;
- les hydrocarbures linéaires ou ramifiés, d'origine minérale ou synthétique tels que les huiles de paraffine et leurs dérivés, l'isoparaffine, l'isohexadécane, le polyisobutène hydrogéné tel que le parléam , les polydécènes ;
- des esters et les éthers de synthèse comme le myristate d'isopropyle, des octanoates, décanoates ou ricinoléates d'alcools ou de polyalcools ;
- des alcools gras comme l'actyl dodécanol ou l'alcool oléique ;
- leurs mélanges.

Les huiles siliconées utilisées selon l'invention en association avec l'organopolysiloxane élastomérique partiellement réticulé sont choisies de préférence parmi les polysiloxanes linéaires liquides ou pâteux à température ambiante tels que méthylpolysiloxane, méthylphénylpolysiloxane, éthylpolysiloxane, éthylméthylpolysiloxane, éthylphénylpolysiloxane, hydroxyméthylpolysiloxane, alkylpolydiméthylsiloxane et les polysiloxanes cycliques tels que octaméthylcyclopentasiloxane, décaméthylcyclopentasiloxane; ou leurs mélanges.

De façon préférentielle, l'organopolysiloxane est présent dans le mélange organopolysiloxane/phase grasse pour former l'agent matifiant sous forme d'un gel plus ou moins homogène dans une concentration allant de 1 à 80 % en poids.

Le gel résultant de cette association peut être utilisé tel quel et constituer lui-même une composition pour le soin ou le maquillage pour matifier la peau et/ou pour estomper les défauts du relief de la peau. Il peut également être incorporé dans une formulation pour le soin ou le maquillage dans une quantité suffisante pour permettre à celle-ci d'apporter à la peau un bon effet matifiant et/ou d'estomper de manière satisfaisante les défauts du relief de la peau.

Les compositions selon l'invention contenant les agents matifiants tels que définis ci-dessus se présentent de préférence sous forme de gels pouvant être translucides ou opaques. Elles peuvent se présenter aussi sous forme d'émulsions huile/eau ou d'émulsions eau/huile pour produire des crèmes matifiantes.

Elles peuvent contenir en plus des adjuvants classiques tels que des colorants hydrosolubles ou liposolubles, des pigments, des parfums, des conservateurs, des filtres solaires, des actifs liposolubles ou hydrosolubles. Ces adjuvants sont présents dans des quantités allant de préférence de 0 à 20% en poids par rapport au poids de la composition.

Elles peuvent contenir en plus des charges pour modifier la texture de la formulation telles que la silice, la poudre de Nylon, la poudre de polyéthylène, la poudre de poly(méthacrylate de méthyle) ou de ses dérivés. Ces charges sont présentes dans des quantités allant de préférence de 0 à 40% en poids par rapport au poids de la composition.

Bien entendu, les adjuvants et les charges introduits dans la composition doivent de nature et en quantité ne nuisant pas à l'effet recherché.

Comme indiqué plus avant, l'effet matifiant de la composition selon l'invention est lié à la diffusion de la lumière par l'organopolysiloxane élastomérique. Grâce à cette diffusion importante de la lumière, le microrelief du support sur lequel est appliqué la composition n'est plus distinctement visible. Cet effet est d'autant plus notable que la composition est transparente. En particulier, la transparence de la composition permet de diminuer les « ombres portées » des ridules et rides, ombres qui normalement accusent le relief des ridules et rides. Ainsi, la transparence de la composition a non seulement un intérêt dans l'effet visuel de la composition mais aussi, après application, dans l'effet obtenu sur le support.

Aussi, un autre objet de l'invention consiste en l'utilisation d'un organopolysiloxane élastomérique partiellement réticulé tel que défini précédemment associé à une phase grasse pour la préparation d'une composition ou dans une composition de soin de la peau ou de maquillage, pour estomper les imperfections du relief de la peau, en particulier pour camoufler les micro-reliefs, les rides et les ridules, les pores.

L'invention concerne également un procédé de traitement non thérapeutique de la peau destiné à lui apporter un aspect mat et/ou à camoufler les défauts du relief de la peau, caractérisé par le fait qu'on applique sur la peau dans une quantité efficace d'une composition contenant au moins un organopolysiloxane élastomérique partiellement réticulé tel que défini précédemment en présence d'une phase grasse.

Les exemples qui suivent servent à illustrer l'invention sans toutefois présenter un caractère limitatif.

<u>**EXEMPLE 1**</u> : Composition matifiante

- Huile de polydiméthylsiloxane 6 cst          30 % en poids
- Polydiméthylorganosiloxane partiellement réticulé vendu sous le nom KSG 6 par SHIN ETSU          20 % en poids
- Triglycérides des acides caprylique/caprique vendus sous les dénominations MIGLYOL par la société DYNAMIT NOBEL          9,1 % en poids
- Huile de parléam          9,1 % en poids
- Silice vendue sous le nom SB150 par la société MAPRECOS          31,8 % en poids

On obtient par simple mélange et homogénéisation des différents ingrédients à température ambiante un gel translucide, doux, facilement étalable et non desséchant. Le gel ainsi obtenu possède des propriétés matifiantes stables dans le temps et donne un aspect naturel une fois appliqué sur la peau.

On mesure l'évolution de la brillance de la peau au cours du temps après 15 minutes, 2 heures, 4 heures et 6 heures, observée sur un échantillon de 4 personnes (2 hommes et 2 femmes), à peau grasse et brillante sur lesquelles on a appliqué la composition matifiante de l'exemple 1.

On applique la composition matifiante sur chaque personne, à raison de 2 mg/cm$^2$ en une application unique sur un demi-front, l'autre moitié servant de zone témoin. Une randomisation est effectuée pour éviter les effets de zone.

Les conditions climatiques sont les suivantes :

Température : 21°C
Humidité relative : 38%

On mesure aux temps T= 0 ($T_0$), T= 15 mn, T=2h, T= 4h, T=6h, la brillance de la surface de la peau maquillée (ou non traitée pour la zone témoin) au moyen d'un appareil de mesure décrit dans la demande publiée FR 2.665.959 et à partir des paramètres de réflexion parallèle et de réflexion croisée propres à ce dispositif et permettant d'évaluer la brillance de la surface de la peau.

On calcule dans un premier temps, au temps T, la variation de la brillance moyenne mesurée sur la zone traitée par la formule :

$$\Delta_1 = B_T - B_O/B_O$$

où :

$B_0$ désigne la brillance moyenne mesurée à $T_0$

$B_T$ désigne la brillance moyenne mesurée à T

On calcule dans un second temps, au temps T, la variation de la brillance moyenne mesurée sur la zone témoin par la formule :

$$\Delta_2 = B'_T - B'_O / B'_O$$

où :

$B'_0$ désigne la brillance moyenne mesurée sur la zone témoin à $T_0$

$B'_T$ désigne la brillance moyenne mesurée sur la zone témoin à T

On détermine enfin le pourcentage d'évolution de la brillance moyenne par la différence $\Delta_1 - \Delta_2$. Ce paramètre permet de déterminer l'effet matifiant de la composition selon l'invention à savoir le pourcentage de diminution de la brillance et la tenue de la matité au cours du temps.

Les résultats de ces tests sont résumés dans le tableau suivant :

| Temps | 15 minutes | 2 heures | 4 heures | 6 heures |
|---|---|---|---|---|
| Variation de la brillance moyenne sur zone traitée $\Delta_1$ | -18 % | -12% | -10% | -9% |
| Variation de la brillance moyenne sur zone témoin $\Delta_2$ | 10% | 11% | 18% | 12% |
| Pourcentage d'évolution de la brillance moyenne $\Delta_1 - \Delta_2$ | -28% | -23% | -28% | -21% |

D'après de tableau, on constate bien que la composition de l'invention matifie de manière très nette après 15 mn (diminution de la brillance de 28%) et présente une bonne tenue de la matité au cours du temps jusqu'à 6 heures (diminution de la brillance de 21%).

**EXEMPLE 2** : Crème matifiante (émulsion huile-dans-eau)

- Mélange de 40% en poids huile de polydiméthylsiloxane 6 cst et de 60% en poids de polydiméthylorganosiloxane partiellement réticulé vendu sous le nom KSG 6 par KOSE        10% en poids
- Tensioactif alkyl diméthicone copolyol vendu sous le nom WE O9 par GOLDSCHMIDT        5% en poids
- Perhydrosqualène        10 % en poids
- Conservateur        qs
- Eau        qsp        100 % en poids

On obtient par simple mélange et homogénéisation des différents ingrédients à température ambiante une crème gélifiée, douce, facilement étalable et non desséchante. La crème ainsi obtenue possède des propriétés matifiantes stables dans le temps et donne un aspect naturel une fois appliquée sur la peau.

**Revendications**

1. Utilisation d'un organopolysiloxane solide élastomérique au moins partiellement réticulé associé à une phase grasse, sous forme de gel comme agent matifiant cosmétique, pouvant être utilisé tel quel ou incorporé comme additif dans une composition de maquillage ou de soin.

2. Utilisation selon la revendication 1, caractérisée par le fait que l'organopolysiloxane est partiellement ou totalement réticulé.

3. Utilisation selon l'une quelconque des revendications 1 ou 2, caractérisée par le fait que l'organopolysiloxane est inclus dans au moins une huile hydrocarbonée et/ou au moins une huile siliconée pour former ledit gel.

**4.** Utilisation selon l'une quelconque des revendications 1 à 3, caractérisée par le fait que l'organopolysiloxane est obtenu par réaction d'addition et de réticulation, en présence d'un catalyseur, d'au moins :

(a) un organopolysiloxane ayant au moins deux groupes alcényle inférieurs par molécule ;
(b) un organopolysiloxane ayant au moins deux atomes d'hydrogène liés à un atome de silicium par molécule.

**5.** Utilisation selon l'une quelconque des revendications 1 à 3, caractérisée par le fait que l'organopolysiloxane est choisi parmi :

i) les organopolysiloxanes comprenant des motifs $R_2SiO$ et $RSiO_{1,5}$ et éventuellement des motifs $R_3SiO_{0,5}$ et/ou $SiO_2$ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs $R_2SiO$ sur les motifs $RSiO_{1,5}$ varie de 1/1 à 30/1 ;

ii) les organopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol quand l'organopolysiloxane est non-cyclique et entre 1 et 50% mol lorsque l'organopolysiloxane est cyclique.

**6.** Utilisation selon l'une quelconque des revendications 1 à 5, caractérisée par le fait que les huiles hydrocarbonées utilisées en association avec l'organopolysiloxane sont choisies parmi :

- les huiles d'origine animale ;
- les huiles végétales ;
- les huiles de formule $R_9COOR_{10}$ dans laquelle $R_9$ représente le reste d'un acide gras supérieur comportant de 7 à 19 atomes de carbone et $R_{10}$ représente une chaîne hydrocarbonée ramifiée contenant de 3 à 20 atomes de carbone, par exemple, l'huile de Purcellin ;
- les hydrocarbures linéaires ou ramifiés d'origine minérale ou synthétique ;
- des esters et ethers de synthèse ;
- leurs mélanges.

**7.** Utilisation selon l'une quelconque des revendications 1 à 6, caractérisée par le fait que les huiles siliconées utilisées en association avec l'organopolysiloxane élastomère sont choisies parmi les polysiloxanes linéaires liquides ou pâteux à température ambiante, les polysiloxanes cycliques ou leurs mélanges.

**8.** Utilisation selon l'une quelconque des revendications 1 à 7, caractérisée par le le fait que l'organopolysiloxane est présent dans le mélange organopolysiloxane élastomérique partiellement réticulé /phase grasse pour former un gel homogène dans une concentration allant de 3 à 80 % en poids.

**Patentansprüche**

**1.** Verwendung eines mindestens teilweise vernetzten, festen Polyorganosiloxans vom Elastomertyp, das mit einer Fettphase kombiniert ist, in Form eines Gels als kosmetisches Mattierungsmittel, das als solches oder als Zusatz in eine Zusammensetzung zum Schminken oder zur Pflege eingebracht verwendet werden kann.

**2.** Verwendung nach Anspruch 1, dadurch gekennzeichnet, daß das Polyorganosiloxan teilweise oder vollständig vernetzt ist.

**3.** Verwendung nach einem der Ansprüche 1 oder 2, dadurch gekennzeichnet, daß das Polyorganosiloxan zur Bildung des Gels in mindestens einem Kohlenwasserstofföl und/oder mindestens einem Siliconöl eingeschlossen ist.

**4.** Verwendung nach einem der Ansprüche 1 bis 3, dadurch gekennzeichnet, daß das Polyorganosiloxan durch Addition und Vernetzung hergestellt wird, wobei mindestens:

- (a) ein Polyorganosiloxan, das pro Molekül mindestens zwei niedere Alkenylgruppen aufweist, und
- (b) ein Polyorganosiloxan, das pro Molekül mindestens zwei Wasserstoffatome aufweist, die an ein Silicium-

**EP 0 790 055 B1**

atom gebunden sind,

in Gegenwart eines Katalysators verwendet werden.

**5.** Verwendung nach einem der Ansprüche 1 bis 3, dadurch gekennzeichnet, daß das Polyorganosiloxan ausgewählt ist unter:

- (i) Polyorganosiloxanen, die Einheiten $R_2SiO$ und $RSiO_{1,5}$ und gegebenenfalls Einheiten $R_3SiO_{0,5}$ und/oder $SiO_2$ aufweisen, worin die Gruppen R unabhängig voneinander Wasserstoff, eine Alkylgruppe, wie Methyl, Ethyl oder Propyl, eine Arylgruppe, wie Phenyl oder Tolyl, oder eine ungesättigte aliphatische Gruppe, wie Vinyl, bedeuten und worin das Gewichtsverhältnis der Einheiten $R_2SiO$ zu den Einheiten $RSiO_{1,5}$ im Bereich von 1/1 bis 30/1 liegt, und
- (ii) Polyorganosiloxanen, die in dem Siliconöl unlöslich und quellfähig sind und die durch Addition eines Polyorganohydrogenosiloxans (1) und eines Polyorganosiloxans (2) mit ungesättigten aliphatischen Gruppen so hergestellt werden, daß die Wasserstoffmenge oder die Menge ungesättigter aliphatischer Gruppen in (1) bzw. (2) im Bereich von 1 bis 20 Mol-% liegt, wenn das Polyorganosiloxan nicht cyclisch vorliegt, und im Bereich von 1 bis 50 Mol-%, wenn das Polyorganosiloxan cyclisch vorliegt.

**6.** Verwendung nach einem der Ansprüche 1 bis 5, dadurch gekennzeichnet, daß die Kohlenwasserstofföle, die in Kombination mit dem Polyorganosiloxan verwendet werden, ausgewählt sind unter:

- Ölen tierischer Herkunft,
- pflanzlichen Ölen,
- Ölen der Formel $R_9COOR_{10}$, worin $R_9$ den Rest einer höheren Fettsäure bedeutet, die 7 bis 19 Kohlenstoffatome aufweist, und worin $R_{10}$ eine verzweigte Kohlenwasserstoffkette bedeutet, die 3 bis 20 Kohlenstoffatome enthält, wie beispielsweise Purcellinöl,
- geradkettigen oder verzweigten Kohlenwasserstoffen mineralischen oder synthetischen Ursprungs,
- synthetischen Estern und Ethern und
- den Gemischen dieser Verbindungen.

**7.** Verwendung nach einem der Ansprüche 1 bis 6, dadurch gekennzeichnet, daß die Siliconöle, die in Kombination mit dem Polyorganosiloxan vom Elastomertyp verwendet werden, unter den geradkettigen Polysiloxanen, die bei Umgebungstemperatur flüssig oder pastös sind, den cyclischen Polysiloxanen oder den Gemischen dieser Verbindungen ausgewählt sind.

**8.** Verwendung nach einem der Ansprüche 1 bis 7, dadurch gekennzeichnet, daß das Polyorganosiloxan in dem Gemisch von teilweise vernetztem Polyorganosiloxan vom Elastomertyp und Fettphase zur Bildung eines homogenen Gels in einer Konzentration im Bereich von 3 bis 80 Gew.-% vorliegt.

**Claims**

**1.** Use of an at least partially crosslinked elastomeric solid organopolysiloxane associated with a fatty phase, in the form of gel as cosmetic matting agent, which can be employed as it is or incorporated as additive into a make-up or care composition.

**2.** Use according to Claim 1, characterized in that the organopolysiloxane is partially or completely crosslinked.

**3.** Use according to either of Claims 1 and 2, characterized in that the organopolysiloxane is enclosed in at least one hydrocarbon oil and/or at least one silicone oil in order to form the said gel.

**4.** Use according to any one of Claims 1 to 3, characterized in that the organopolysiloxane is obtained by addition and crosslinking reaction, in the presence of a catalyst, of at least:

(a) an organopolysiloxane containing at least two lower alkenyl groups per molecule;
(b) an organopolysiloxane containing at least two hydrogen atoms bonded to a silicon atom per molecule.

**5.** Use according to any one of Claims 1 to 3, characterized in that the organopolysiloxane is chosen from:

7

i) organopolysiloxanes containing $R_2SiO$ and $RSiO_{1.5}$ units and optionally $R_3SiO_{0.5}$ and/or $SiO_2$ units, in which the radicals R, independently of one another, denote a hydrogen, an alkyl such as methyl, ethyl or propyl, an aryl such as phenyl or tolyl or an unsaturated aliphatic group such as vinyl, and where the weight ratio of the $R_2SiO$ units to the $RSiO_{1.5}$ units varies from 1/1 to 30/1;

ii) organopolysiloxanes which are insoluble and swellable in silicone oil, which are obtained by addition of an organohydropolysiloxane (1) and of an organopolysiloxane (2) containing unsaturated aliphatic groups, such that the quantity of hydrogen or of unsaturated aliphatic groups in (1) and (2) respectively is between 1 and 20 mol% when the organopolysiloxane is noncyclic and between 1 and 50 mol% when the organopolysiloxane is cyclic.

6.   Use according to any one of Claims 1 to 5, characterized in that the hydrocarbon oils employed in association with the organopolysiloxane are chosen from:

-   oils of animal origin;
-   vegetable oils;
-   oils of formula $R_9COOR_{10}$ in which $R_9$ denotes the residue of a higher fatty acid containing from 7 to 19 carbon atoms and $R_{10}$ denotes a branched hydrocarbon chain containing from 3 to 20 carbon atoms, for example purcellin oil;
-   linear or branched hydrocarbons of mineral or synthetic origin;
-   synthetic esters and ethers;
-   mixtures thereof.

7.   Use according to any one of Claims 1 to 6, characterized in that the silicone oils employed in association with the organopolysiloxane are chosen from linear polysiloxanes which are liquid or pasty at ambient temperature, cyclic polysiloxanes or mixtures thereof.

8.   Use according to any one of Claims 1 to 7, characterized in that the organopolysiloxane is present in the partially crosslinked elastomeric organopolysiloxane/fatty phase mixture in order to form a homogeneous gel in a concentration ranging from 3 to 80 % by weight.